(19) Europäisches Patentamt
European Patent Office
Office européen des brevets

(11) Veröffentlichungsnummer: **0 362 752 A1**

(12) **EUROPÄISCHE PATENTANMELDUNG**

(21) Anmeldenummer: **89118201.6**

(22) Anmeldetag: **30.09.89**

(51) Int. Cl.5: **C07D 307/46 , C07D 333/22 , C07D 207/333 , A01N 43/08 , A01N 43/10 , A01N 43/36**

(30) Priorität: **05.10.88 DE 3834249**

(43) Veröffentlichungstag der Anmeldung: **11.04.90 Patentblatt 90/15**

(84) Benannte Vertragsstaaten: **AT BE CH DE ES FR GB GR IT LI LU NL SE**

(71) Anmelder: **SCHERING AKTIENGESELLSCHAFT**
**Berlin und Bergkamen**
**Müllerstrasse 170/178 Postfach 65 03 11**
**D-1000 Berlin 65(DE)**

(72) Erfinder: **Angermann, Alfred, Dr.**
**Nussbaumallee 12**
**D-1000 Berlin 19(DE)**
Erfinder: **Franke, Helga, Dr.**
**Spiessergasse 6b**
**D-1000 Berlin 27(DE)**
Erfinder: **Bohner, Jürgen, Dr.**
**Germendorfer Strasse 57**
**D-1000 Berlin 26(DE)**

(54) **3,5-Dioxo-4-heteroaroylcyclohexancarbonsäurederivate, Verfahren zur Herstellung dieser Verbindungen und ihre Verwendung als Mittel mit pflanzenwachstumsregulierender Wirkung.**

(57) 3,5-Dioxo-4-heteroaroylcyclohexancarbonsäurederivate der allgemeinen Formel

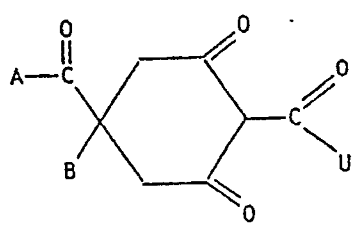

sowie die daraus abgeleiteten Salze, Formel in der U einen Furyl-, Thienyl- oder Pyrrylrest, und A einen Hydroxy-, Alkoxy- oder Aminorest bedeuten, ihre Herstellung ind ihre Verwendung als Mittel mit pflanzenwachstumsregulierender Wirkung.

EP 0 362 752 A1

# 3,5-DIOXO-4-HETEROAROYLCYCLOHEXANCARBONSÄUREDERIVATE, VERFAHREN ZUR HERSTELLUNG DIESER VERBINDUNGEN UND IHRE VERWENDUNG ALS MITTEL MIT PFLANZENWACHSTUMSREGULIERENDER WIRKUNG

Die Erfindung betrifft neue 3,5-Dioxo-4-heteroaroylcyclohexancarbonsäurederivate, Verfahren zur Herstellung dieser Verbindungen und ihre Verwendung als Mittel mit pflanzenwachstumsregulierender Wirkung.

Es ist bereits bekannt, daß gewisse Cyclohexandioncarbonsäurederivate als Pflanzenwachstumsregulatoren eingesetzt werden können (EP-Anmeldungen 0 123 001 und 0 126 713). Bei diesen bekannten Verbindungen ist die pflanzenwachstumsregulierende Wirkung aber nicht immer ausreichend oder es treten phytotoxische Nebenwirkungen bei den behandelten Kulturpflanzen auf.

Aufgabe der vorliegenden Erfindung ist die Bereitstellung von neuen Verbindungen, die in ihren biologischen Eigenschaften den bisher bekannten Verbindungen überlegen sind.

Diese Aufgabe wird gelöst durch neue 3,5-Dioxo-4-heteroaroylcyclohexancarbonsäurederivate der allgemeinen Formel I

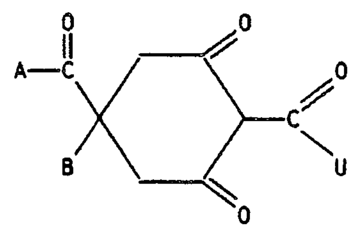

(I) ,

in der

A eine der Gruppen -OM, $-OR^1$ oder $-NR^2R^3$,

B ein Wasserstoffatom, einen $C_1$-$C_8$-Alkylrest, einen $C_2$-$C_8$-Alkenylrest, einen $C_2$-$C_8$-Alkinylrest, einen Halogen-$C_1$-$C_8$-Alkylrest, einen Halogen-$C_2$-$C_8$-Alkenylrest oder einen Halogen-$C_2$-$C_8$-Alkinylrest,

M ein Kation aus der Gruppe Lithium, Natrium und Kalium, ein Äquivalent aus der Gruppe Zink, Mangan, Calcium, Magnesium und Barium oder ein Ammoniumion der allgemeinen Formel

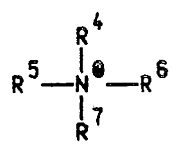

$R^1$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein- oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano oder $C_1$-$C_4$-Alkyl substituierten Phenylrest, einen Phenyl-$C_1$-$C_2$-alkylrest oder einen ein- oder mehrfach, gleich oder verschieden durch Halogen, Nitro, Cyano oder $C_1$-$C_4$-Alkyl substituierten Phenyl-$C_1$-$C_2$-alkylrest,

$R^2$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein- oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch halogen, Nitro, Cyano oder $C_1$-$C_4$-Alkyl substituierten Phenylrest, einen Phenyl-$C_1$-$C_2$-alkylrest oder einen ein- oder mehrfach, gleich oder verschieden durch Halogen, Nitro, Cyano oder $C_1$-

$C_4$-Alkyl substituierten Phenyl-$C_1$-$C_2$-alkylrest,

$R^3$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein-oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano oder $C_1$-$C_4$-Alkyl substituierten Phenylrest, einen Phenyl-$C_1$-$C_2$-alkylrest oder einen ein- oder mehrfach, gleich oder verschieden durch Halogen, Nitro, Cyano oder $C_1$-$C_4$-Alkyl substituierten Phenyl-$C_1$-$C_2$-alkylrest oder

$R^2$ und $R^3$ gemeinsam mit dem benachbarten Stickstoffatom eine Morpholinogruppe, eine Piperidinogruppe oder eine Pyrrolidinogruppe,

$R^4$ ein Wasserstoffatom, einen $C_1$-$C_{18}$-Alkylrest, einen ein- oder mehrfach, gleich oder verschieden durch Halogen, Hydroxy, Nitro, Cyano oder $C_1$-$C_6$-Alkoxy substituierten $C_1$-$C_{18}$-Alkylrest, einen Phenylrest, einen ein-oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenyl-$C_1$-$C_2$-alkylrest,

$R^5$ ein Wasserstoffatom, einen $C_1$-$C_{18}$-Alkylrest, einen ein- oder mehrfach, gleich oder verschieden durch Halogen, Hydroxy, Nitro, Cyano oder $C_1$-$C_6$-Alkoxy substituierten $C_1$-$C_{18}$-Alkylrest, einen Phenylrest, einen ein-oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenyl-$C_1$-$C_2$-alkylrest,

$R^6$ ein Wasserstoffatom, einen, $C_1$-$C_{18}$-Alkylrest, einen ein- oder mehrfach, gleich oder verschieden durch Halogen, Hydroxy, Nitro, Cyan oder $C_1$-$C_6$-Alkoxy substituierten $C_1$-$C_{18}$-Alkylrest, einen Phenylrest, einen ein-oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenyl-$C_1$-$C_2$-alkylrest,

$R^7$ ein Wasserstoffatom, einen $C_1$-$C_{18}$-Alkylrest, einen ein- oder mehrfach, gleich oder verschieden durch Halogen, Hydroxy, Nitro, Cyano oder $C_1$-$C_6$-Alkoxy substituierten $C_1$-$C_{18}$-Alkylrest, einen Phenylrest, einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenyl-$C_1$-$C_2$-alkylrest,

U eine der Gruppen U - 1 oder U - 2

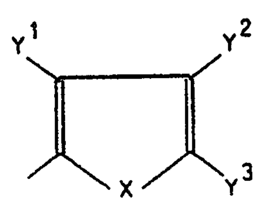

U - 1

oder

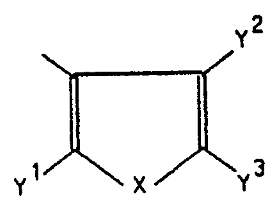

U - 2

,

X ein Sauerstoffatom, ein Schwefelatom oder eine Gruppe N-$R^8$,

$R^8$ ein Wasserstoffatom oder einen $C_1$-$C_4$-Alkylrest,

$Y^1$ ein Wasserstoffatom, einen $C_1$-$C_8$-Alkylrest, einen durch Halogen, $C_1$-$C_8$-Alkoxy, Hydroxy oder Mercapto substituierten $C_1$-$C_8$-Alkylrest, einen $C_2$-$C_8$-Alkenylrest, einen durch Halogen, $C_1$-$C_8$-Alkoxy, Hydroxy oder Mercapto substituierten $C_2$-$C_8$-Alkenylrest, einen $C_2$-$C_8$-Alkinylrest, einen durch Halogen, $C_1$-$C_8$-Alkoxy, Hydroxy oder Mercapto substituierten $C_2$-$C_8$-Alkinylrest, einen $C_1$-$C_8$-Alkoxyrest, einen Halogen-$C_1$-$C_8$-Alkoxyrest, ein Halogenatom, einen Nitrorest, einen Cyanorest, einen Trimethylsilylrest oder eine der Gruppen -COA, -$COR^1$, -$PO(OR^1)_2$ oder -$S(O)_mR^1$,

$Y^2$ ein Wasserstoffatom, einen $C_1$-$C_8$-Alkylrest, einen durch Halogen, $C_1$-$C_8$-Alkoxy, Hydroxy oder Mercapto substituierten $C_1$-$C_8$-Alkylrest, einen $C_2$-$C_8$-Alkenylrest, einen durch Halogen, $C_1$-$C_8$-Alkoxy, Hydroxy oder

Mercapto substituierten $C_2$-$C_8$-Alkenylrest, einen $C_2$-$C_8$-Alkinylrest, einen durch Halogen, $C_1$-$C_8$-Alkoxy, Hydroxy oder Mercapto substituierten $C_2$-$C_8$-Alkinylrest, einen $C_1$-$C_8$-Alkoxyrest, einen Halogen-$C_1$-$C_8$-Alkoxyrest, ein Halogenatom, einen Nitrorest, einen Cyanorest, einen Trimethylsilylrest oder einen der Gruppen -COA, -COR[1], -PO(OR[1])$_2$ oder -S(O)$_m$R[1],

$Y^3$ ein Wasserstoffatom, einen $C_1$-$C_8$-Alkylrest, einen durch Halogen, $C_1$-$C_8$-Alkoxy, Hydroxy oder Mercapto substituierten $C_1$-$C_8$-Alkylrest, einen $C_2$-$C_8$-Alkenylrest, einen durch Halogen, $C_1$-$C_8$-Alkoxy, Hydroxy oder Mercapto substituierten $C_2$-$C_8$-Alkenylrest, einen $C_2$-$C_8$-Alkinylrest, einen durch Halogen, $C_1$-$C_8$-Alkoxy, Hydroxy oder Mercapto substituierten $C_2$-$C_8$-Alkinylrest, einen $C_1$-$C_8$-Alkoxyrest, einen Halogen-$C_1$-$C_8$-Alkoxyrest, ein Halogenatom, einen Nitrorest, einen Cyanorest, einen Trimethylsilylrest oder eine der Gruppen -COA, -COR[1], -PO(OR[1])$_2$ oder -S(O)$_m$R[1] und

m eine Zahl 0, 1 oder 2

bedeuten, sowie die daraus abgeleiteten Salze der allgemeinen Formel II

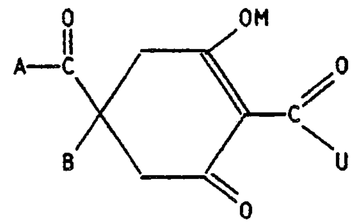

(II) ,

in der A, B, M und U die unter der allgemeinen Formel I genannten Bedeutungen haben.

Unter den Begriffen "Alkyl", "Alkenyl" und "Alkinyl" sind sowohl geradkettige als auch verzweigte Kohlenwasserstoffreste zu verstehen. Die Bezeichnungen "Halogenalkyl", "Halogenalkenyl" und "Halogenalkinyl" bedeuten, daß ein oder mehrere Wasserstoffatome des Kohlenwasserstoffrestes durch Halogen ersetzt sind. Die Bezeichnung "Halogen" umfaßt Fluor, Chlor, Brom und Jod.

Die erfindungsgemäßen Verbindungen der allgemeinen Formel I können auch als Tautomere mit den allgemeinen Formeln I′ und I″ vorliegen

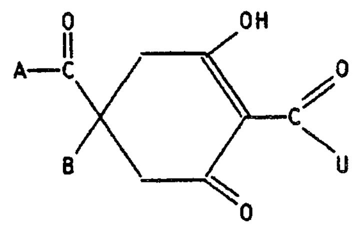

(I′) ,

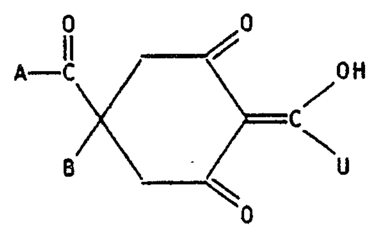

(I″) ,

in denen A, B und U die unter der allgemeinen Formel I genannten Bedeutungen haben. Diese Strukturen werden ebenfalls durch die vorliegende Erfindung umfaßt, aus Gründen der Einfachheit wird aber jeweils nur die Struktur der allgemeinen Formel I angegeben.

Die erfindungsgemäßen Verbindungen der allgemeinen Formel I lassen sich herstellen, indem man eine Verbindung der allgemeinen Formel III

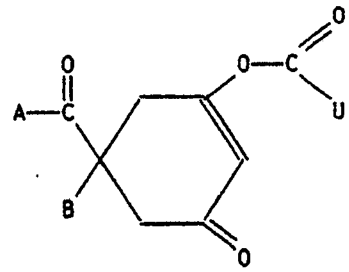

(III) ,

in der A, B und U die unter der allgemeinen Formel I angegebenen Bedeutungen haben, einer katalytisch geförderten Umlagerungsreaktion unterwirft.

Als Katalysatoren für diese Umlagerungsreaktion eignen sich Pyridinderivate, wie zum Beispiel 4-(N,N-Dialkylamino)-pyridine, vorzugsweise 4-(N,N-Dimethylamino)-pyridin; N-Alkylimidazole, wie zum Beispiel N-Methylimidazol; Metallcyanide, wie zum Beispiel Kupfer-I-cyanid, Natriumcyanid, Kaliumcyanid und Zinkcyanid, 2-Hydroxyisobutansäurenitril (Acetoncyanhydrin) oder Lewis-Säuren, wie zum Beispiel Zinkchlorid oder Aluminiumchlorid.

Die Umsetzungen werden unter Feuchtigkeitsausschluß und gegebenenfalls in Anwesenheit eines inerten Lösungsmittels durchgeführt. Als besonders geeignet haben sich hierfür aromatische Kohlenwasserstoffe, wie Benzol, Toluol und Xylol; Halogenkohlenwasserstoffe, wie zum Beispiel Tetrachlorkohlenstoff, Trichlormethan und Dichlormethan; Acetonitril und organische Basen, wie Triethylamin und Pyridin, erwiesen.

Die Ausgangsstoffe der allgemeinen Formel III erhält man durch Acylierung eines 3,5-Dioxocyclohexancarbonsäurederivates der allgemeinen Formel IV

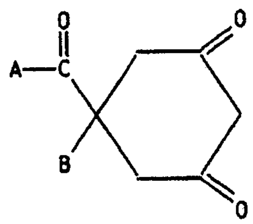

(IV) ,

in der A und B die unter der allgemeinen Formel I genannten Bedeutungen haben, mit Reagenzien der allgemeinen Formeln V oder VI

$$Z - \overset{O}{\overset{\|}{C}} - U \qquad (V)$$

oder

$$U - \overset{O}{\overset{\|}{C}} - O - \overset{O}{\overset{\|}{C}} - U \qquad (VI) ,$$

in denen U die unter der allgemeinen Formel I angegebene Bedeutung hat und Z für eine Fluchtgruppe, wie zum Beispiel Halogen, vorzugsweise für ein Chlor- oder Bromatom, steht. Solche Verbindungen der allgemeinen Formel V und VI sind entweder literaturbekannt oder können in Anlehnung an literaturbekannte Methoden hergestellt werden.

Die Acylierungsreaktion wird gegebenenfalls in Anwesenheit eines inerten Lösungsmittels und eines säurebindenden Mittels durchgeführt. Als solche säurebindenden Mittel kommen verschiedene organische und anorganische Basen in Betracht, als besonders geeignet haben sich jedoch Triethylamin und Pyridin erwiesen.

Bei den beschriebenen Reaktionen können - neben der bereits erwähnten -auch alle solchen Lösungsbeziehungsweise Verdünnungsmittel zum Einsatz kommen, die gegenüber den jeweiligen Reaktanden inert sind. Beispiele für solche Lösungsmittel beziehungssweise Verdünnungsmittel sind aliphatische, alicyclische und aromatische Kohlenwasserstoffe, die jeweils gegebenenfalls chloriert sein können, wie zum

Beispiel Hexan, Cyclohexan, Petrolether, Ligroin, Benzol, Toluol, Xylol, Methylenchlorid, Chloroform, Kohlenstofftetrachlorid, Ethylenchlorid, Trichlorethylen und Chlorbenzol; Ether, wie zum Beispiel Diethylether, Methylethylether, Methyl-t-butylether, Diisopropylether, Dibutylether, Dioxan und Tetrahydrofuran; Ketone, wie zum Beispiel Aceton, Methylethylketon, Methylisopropylketon und Methylisobutylketon; Nitrile, wie zum Beispiel Acetonitril und Propionitril; Alkohole, wie zum Beispiel Methanol, Ethanol, Isopropanol, Butanol und Ethylenglycol; Ester, wie zum Beispiel Ethylacetat und Amylacetat; Säureamide, wie zum Beispiel Dimethylformamid und Dimethylacetamid; Sulfoxide und Sulfone, wie zum Beispiel Dimethylsulfoxid und Sulfolan; sowie Basen, wie zum Beispiel Pyridin.

Alle Reaktion werden vorzugsweise unter dem Druck der Umgebung durchgeführt, wenngleich sie auch bei erhöhtem oder vermindertem Druck durchgeführt werden könnten.

Die Reaktionen können ferner innerhalb eines breiten Temperaturbereichs durchgeführt werden. Im allgemeinen werden sie bei einer Temperatur zwischen -20°C und dem Siedepunkt des Reaktionsgemisches, vorzugsweise bei 0°C bis 150°C, durchgeführt.

Die Gegenwart eines zusätzlichen Reaktionskatalysators kann von Vorteil sein. Als Katalysatoren sind Kaliumiodid und Oniumverbindungen geeignet, wie quarternäre Ammonium-, Phosphonium- und Arsoniumsowie Sulfoniumverbindungen.

Ebenfalls geeignet sind Polyglycolether, insbesondere cyclische, wie zum Beispiel 18-Krone-6, und tertiäre Amine, wie zum Beispiel Tributylamin. Bevorzugt sind quarternäre Ammoniumverbindungen, wie zum Beispiel Benzyltriethylammoniumbromid und Tetrabutylammoniumbromid.

Die nach dem oben genannten Verfahren hergestellten erfindungsgemäßen Verbindungen können nach den üblichen Methoden aus dem Reaktionsgemisch isoliert werden, beispielsweise durch Abdestillieren des eingesetzten Lösungsmittels bei normalem oder vermindertem Druck, durch Ausfällen mit Wasser oder durch Extraktion. Ein erhöhter Reinheitsgrad kann in der Regel durch säulenchromatographische Aufreinigung sowie durch fraktionierte Destillation oder Kristallisation erhalten werden.

Die erfindungsgemäßen Verbindungen stellen in der Regel fast farb- und geruchlose Flüssigkeiten sowie Kristalle dar, die bedingt löslich in Wasser, aliphatischen Kohlenwasserstoffen, wie Petrolether, Hexan, Pentan und Cyclohexan, gut löslich in halogenierten Kohlenwasserstoffen, wie Chloroform, Methylenchlorid und Tetrachlorkohlenstoff; aromatischen Kohlenwasserstoffen, wie Benzol, Toluol und Xylol; Ethern, wie Diethylether, Tetrahydrofuran und Dioxan; Carbonsäurenitrilen, wie Acetonitril; Alkoholen, wie Methanol und Ethanol; Carbonsäureamiden, wie Dimethylformamid; Sulfoxiden, wie Dimethylsulfoxid, und - aufgrund ihres in der Regel sauren Charakters -in Basen wie Alkalihydroxid-, Alkalicarbonat- und Alkalihydrogencarbonatlösungen, Triethylamin und Pyridin sind.

Die erfindungsgemäßen Verbindungen verursachen sowohl qualitative wie quantitative Veränderungen von Pflanzen als auch Veränderungen im Metabolismus der Pflanzen und sind daher in die Klasse der Pflanzenwachstumsregulatoren einzustufen, die sich durch folgende Anwendungsmöglichkeiten auszeichnen:

- Hemmung des vegetativen Wachstums bei holzigen und krautigen Pflanzen zum Beispiel an Straßenrändern, Gleisanlagen und anderen, um ein zu üppiges Wachstum zu unterbinden. Wuchshemmung beim Getreide, um das Lagern oder Umknicken zu unterbinden, bei Baumwolle zur Ertragserhöhung.
- Beeinflussung der Verzweigung von vegetativen und generativen Organen bei Zier- und Kulturpflanzen zur Vermehrung des Blütenansatzes oder bei Tabak und Tomate zur Hemmung von Seitentrieben.
- Verbesserung der Fruchtqualität, zum Beispiel eine Zuckergehaltssteigerung beim Zuckerrohr, bei Zuckerrüben oder bei Obst und eine gleichmäßigere Reife des Erntegutes, die zu höheren Erträgen führt.
-Erhöhung der Widerstandskraft gegen Streß, so zum Beispiel gegen klimatische Einflüsse, wie Kälte und Trockenheit, aber auch gegen phytotoxische Einflüsse von Chemikalien.
- Beeinflussung des Latexflusses bei Gummipflanzen.
- Ausbildung parthenokarper Früchte, Pollensterilität und Geschlechtsbeeinflussung sind ebenfalls Anwendungsmöglichkeiten.
- Kontrolle der Keimung von Samen oder des Austriebs von Knospen.
- Entlaubung oder Beeinflussung des Fruchtfalles zur Ernteerleichterung.

Die erfindungsgemäßen Verbindungen eignen sich insbesondere zur Hemmung des vegetativen Wachstums bei verschiedenen Pflanzenarten. Eine solche Wuchshemmung wird bisher in der Praxis mit Substanzen verursacht, die entweder nicht konsistent wirken oder in ihrer Wirkung schächer sind als die erfindungsgemäßen Verbindungen.

Dabei handelt es sich zum Beispiel um Stoffe für verschiedene Getreidearten, die eingesetzt werden, um eine Halmverkürzung zu erreichen und der Pflanze eine größere Standfestigkeit zu verleihen. Darüber hinaus können mit einer derartigen Wirkung Pflanzen im Wuchs gehemmt werden, deren Wachstum an bestimmten Standorten nicht erwünscht ist, so zum Beispiel an Straßenrändern, an Gleisanlagen oder auf

Flugplätzen. Auch Grasarten im Rasen können beeinflußt werden, so daß seltener geschnitten werden muß. Wuchshemmende Effekte werden mit solchen Substanzen im allgemeinen auch bei Kulturpflanzen erzielt, die sich durch den Austrieb ruhender Knospen zu intensiv verzweigen, wie zum Beispiel Tabak.

Bei Baumwolle können Wuchshemmungen zu einem gleichmäßigen Ausreifen der Kapseln und dadurch zu höheren Erträgen führen.

Bei Obstbäumen führt die gezielte Beeinflussung des vegetativen Wachstums zu Ertragssteigerungen und arbeitswirtschaftlichen Vorteilen.

Bei Zierpflanzen erreicht man durch eine Wuchsreduktion oft, daß mehr Pflanzen pro Stellfläche angezogen werden können.

Die erfindungsgemäßen Verbindungen entfalten ihre Wirkung sowohl bei Vorals auch bei Nachauflaufbehandlung. Die Aufwendmengen betragen je nach Anwendungsziel 0,001 bis 5 kg/ha, gegebenenfalls können auch höhere Aufwandmengen eingesetzt werden.

Die Anwendungszeit richtet sich nach dem Anwendungziel und den klimatischen Bedingungen.

Zweckmäßig werden die gekennzeichneten Wirkstoffe oder deren Mischungen in Form von Zubereitungen wie Pulvern, Streumitteln, Granulaten, Lösungen, Emulsionen oder Suspensionen, unter Zusatz von flüssigen und/oder festen Trägerstoffen beziehungsweise Verdünnungsmitteln und gegebenenfalls Haft-, Netz, Emulgier- und/oder Dispergierhilfsmitteln angewandt.

Geeignete flüssige Trägerstoffe sind zum Beispiel aliphatische und aromatische Kohlenwasserstoffe wie Benzol, Toluol, Xylol, Cyclohexanon, Isophoron, Dimethylsulfoxid, Dimethylformamid, weiterhin Mineralölfraktionen und Pflanzenöle.

Als feste Trägerstoffe eignen sich Mineralien, zum Beispiel Bentonit, Silicagel, Talkum, Kaolin, Attapulgit, Kalkstein, und pflanzliche Produkte, zum Beispiel Mehle.

An oberflächenaktiven Stoffen sind zu nennen zum Beispiel Calciumligninsulfonat, Polyethylenalkylphenylether, Naphthalinsulfonsäuren und deren Salze, Phenolsulfonsäuren und deren Salze, Formaldehydkondensate, Fettalkoholsulfate sowie substituierte Benzolsulfonsäuren und deren Salze.

Der Anteil des beziehungsweise der Wirkstoffe(s) in den verschiedenen Zubereitungen kann in weiten Grenzen variieren. Beispielsweise enthalten die Mittel etwa 10 bis 90 Gewichtsprozent Wirkstoff, etwa 90 bis 10 Gewichtsprozent flüssige oder feste Trägerstoffe sowie gegebenenfalls bis zu 20 Gewichtsprozent oberflächenaktive Stoffe.

Die Ausbringung der Mittel kann in üblicher Weise erfolgen, zum Beispiel mit Wasser als Träger in Spritzbrühmengen etwa 100 bis 1000 Liter/ha. Eine Anwendung der Mittel im sogenannten Low-Volume und Ultra-Low-Volume-Verfahren ist ebenso möglich wie ihre Applikation in Form von sogenannten Mikrogranulaten.

Die Herstellung dieser Zubereitungen kann in an sich bekannter Art und Weise, zum Beispiel durch Mahl- oder Mischverfahren, durchgeführt werden. Gewünschtenfalls können Zubereitungen der Einzelkomponenten auch erst kurz vor ihrer Verwendung gemischt werden, wie es zum Beispiel im sogenannten Tankmixverfahren in der Praxis durchgeführt wird.

Zur Herstellung der verschiedenen Zubereitungen werden zum Beispiel die folgenden Bestandteile eingesetzt:

A) **Spritzpulver**

1.) 20 Gewichtsprozent Wirkstoff
68 Gewichtsprozent Kaolin
10 Gewichtsprozent Calciumsalz der Ligninsulfonsäure
2 Gewichtsprozent Dialkylnaphthalinsulfonat

2.) 40 Gewichtsprozent Wirkstoff
25 Gewichtsprozent Kaolin
25 Gewichtsprozent kolloidale Kieselsäure
8 Gewichtsprozent Calciumsalz der Ligninsulfonsäure
2 Gewichtsprozent Natriumsalz des N-Methyl-N-oleyl-taurins

B) **Emulsionskonzentrat**

20 Gewichtsprozent Wirkstoff
75 Gewichtsprozent Isophoron
2 Gewichtsprozent ethoxyliertes Rizinusöl
3 Gewichtsprozent Calciumsalz der Dodecylphenylsulfonsäure

Die folgenden Beispiele erläutern die Herstellung der erfindungsgemäßen Verbindungen:

**Beispiel 1**

3,5-Dioxo-4-(5-methyl-2-furoyl)-cyclohexancarbonsäureethylester

Zu 8,00 g (43,4 mmol) 3,5-Dioxocyclohexancarbonsäureethylester, gelöst in 50 ml Dichlormethan und 20 ml Triethylamin werden bei Eistemperatur langsam 8,2 g (57 mmol) 5-Methyl-2-furancarbonsäurechlorid getropft und anschließend noch 3 Stunden bei Raumtemperatur nachgerührt. Man gießt auf Eis, trennt ab und wäscht die organische Phase mit 2 N Salzsäure, gesättigter Bicarbonatlösung und Wasser, trocknet (Magnesiumsulfat) und engt ein. Der so erhaltene 5-(5-Methyl-2-furoyloxy)-3-oxo-4-cyclohexencarbonsäureethylester wird in 100 ml Acetonitril, 10 ml Triethylamin und 1,5 ml Acetoncyanhydrin 24 Stunden bei Raumtemperatur gerührt. Der Ansatz wird in 100 ml Diethylether aufgenommen, mit 4 N Salzsäure gewaschen und zweimal mit je 50 ml 5%iger Kaliumcarbonatlösung extrahiert. Nach Überschichten dieser alkalischen Phase mit 100 ml Diethylether säuert man mit 4 N Salzsäure bis pH 3 an, trennt ab, wäscht mit Wasser, trocknet (Magnesiumsulfat) und rotiert ein. Säulenchromatographie an Kieselgel (Hexan/Dichlormethan) liefert die gewünschte Substanz in Form farbloser Nadeln.
Ausbeute: 7,72 g = 60,8 % der Theorie
Fp.: 87-88°C

Analog werden die folgenden erfindungsgemäßen Verbindungen hergestellt:

| Beispiel | Name der Verbindung | Physikalische Konstante |
|---|---|---|
| 2 | 3,5-Dioxo-4-(2-furoyl)-cyclohexancarbonsäureethylester | Fp.: 30°C |
| 3 | 3,5-Dioxo-4-(3-furoyl)-cyclohexancarbonsäureethylester | Fp.: 37°C |
| 4 | 3,5-Dioxo-4-(3-furoyl)-cyclohexancarbonsäuremethylester | Fp.: 140°C |
| 5 | 3,5-Dioxo-4-(2-methyl-3-furoyl)-cyclohexancarbonsäureethylester | $n_D^{20}$ : 1.5530 |
| 6 | 3,5-Dioxo-4-(2-trimethylsilyl-3-furoyl)-cyclohexancarbonsäureethylester | $n_D^{20}$ : 1.5403 |
| 7 | 3,5-Dioxo-4-(2-thenoyl)-cyclohexancarbonsäureethylester | Fp.: 57°C |
| 8 | 3,5-Dioxo-4-(3-thenoyl)-cyclohexancarbonsäureethylester | Fp.: 39°C |
| 9 | 3,5-Dioxo-4-(3-furoyl)-cyclohexancarbonsäure-n-propylester | Fp.: 43-46°C |
| 10 | 3,5-Dioxo-4-(3-furoyl)-cyclohexancarbonsäureisopropylester | Fp.: 38-40°C |
| 11 | 3,5-Dioxo-4-(3-furoyl)-cyclohexancarbonsäure-n-butylester | $n_D^{20}$: 1,5437 |
| 12 | 3,5-Dioxo-4-(3-furoyl)-cyclohexancarbonsäureisobutylester | Fp.: 37-38°C |

| Beispiel | Name der Verbindung | Physikalische Konstante |
|---|---|---|
| 13 | 3,5-Dioxo-4-(3-furoyl)-cyclohexancarbonsäureallylester | Fp.: 45-47°C |
| 14 | 3,5-Dioxo-4-(3-furoyl)-cyclohexancarbonsäure, Calciumsalz | Fp.: > 240°C |
| 15 | 3,5-Dioxo-4-(3-furoyl)-cyclohexancarbonsäure, Magnesiumsalz | Fp.: > 240°C |
| 16 | 4-(2,5-Dimethyl-3-furoyl)-3,5-dioxocyclohexancarbonsäureethylester | $n_D^{20}$: 1,3172 |
| 17 | 4-(2,5-Dimethyl-3-furoyl)-3,5-dioxocyclohexancarbonsäuremethylester | $n_D^{20}$: 1,5688 |
| 18 | 3,5-Dioxo-4-(3-furoyl)-cyclohexancarbonsäurehexadecylester | Fp.: 47-49°C |
| 19 | 4-(3-Chlor-2-thenoyl)-3,5-dioxocyclohexancarbonsäureethylester | $n_D^{20}$: 1,5341 |
| 20 | 4-(5-Chlor-2-thenoyl)-3,5-dioxocyclohexancarbonsäureethylester | Fp.: 44-46°C |
| 21 | 3,5-Dioxo-4-(5-nitro-2-furoyl)-cyclohexancarbonsäurethylester | Fp.: 110-112°C |

Die folgenden Beispiele erläutern die Anwendungsmöglichkeiten der erfindungsgemäßen Verbindungen:

Beispiel A: **Wuchshemmung bei Wasserreis**

Reispflanzen wurden in Plastiktöpfen bis zum 2- bis 3-Blattstadium angezogen und mit den erfindungsgemäßen Verbindungen behandelt. Die Applikation erfolgte in das Wasser. 18 Tage nach der Behandlung wurde die Pflanzenhöhe bis zum Ansatz des obersten vollentwickelten Blattes gemessen. Die so ermittelten Daten wurden in Relation zur Kontrolle gesetzt und die Wuchshemmung nach folgendem Schema gewertet:
0 = > 90 % der Wuchshöhe der unbehandelten Kontrolle
1 = 81 - 90 % der Wuchshöhe der unbehandelten Kontrolle
2 = 71 - 80 % der Wuchshöhe der unbehandelten Kontrolle
3 = 61 - 70 % der Wuchshöhe der unbehandelten Kontrolle
4 = < 61 % der Wuchshöhe der unbehandelten Kontrolle
Die Ergebnisse sind in der nachfolgenden Tabelle zusammengestellt.

| Erfindungsgemäße Verbindung | Aufwandmenge (kg/ha) | Wuchshemmung |
|---|---|---|
| Beispiel 4 | 1,0 | 3 |
| | 3,0 | 4 |
| Beispiel 5 | 1,0 | 4 |
| | 3,0 | 4 |
| Beispiel 8 | 1,0 | 2 |
| | 3,0 | 3 |
| Beispiel 9 | 1,0 | 2 |
| | 3,0 | 3 |

| Erfindungsgemäße Verbindung | Aufwandmenge (kg/ha) | Wuchshemmung |
|---|---|---|
| Beispiel 10 | 1,0 | 2 |
| | 3,0 | 3 |
| Beispiel 11 | 1,0 | 3 |
| | 3,0 | 3 |
| Beispiel 12 | 1,0 | 2 |
| | 3,0 | 3 |
| Beispiel 13 | 1,0 | 3 |
| | 3,0 | 4 |

Beispiel B: **Wuchshemmung bei verschiedenen Kulturpflanzen**

Reis und Gerste wurden in humusreicher Erde bis zum 2-Blattstadium angezogen. Danach erfolgte eine Spritzapplikation der Wirkstoffe in Aufwandmengen bis zu 1,0 kg Wirkstoff/ha. 18 Tage nach der Behandlung wurde die Länge der Sprossachse von der Bodenoberfläche bis zum Ansatz des obersten voll entwichelten Blattes gemessen. Die so ermittelten Daten wurden in Relation zur Kontrolle gesetzt und die Wuchshemmung nach folgendem Schema bewertet:
0 = > 90 % der Wuchshöhe der unbehandelten Kontrolle
1 = 81 - 90 % der Wuchshöhe der unbehandelten Kontrolle
2 = 71 - 80 % der Wuchshöhe der unbehandelten Kontrolle
3 = 61 - 70 % der Wuchshöhe der unbehandelten Kontrolle
4 = < 61 % der Wuchshöhe der unbehandelten Kontrolle

Die Ergebnisse sind in der nachfolgenden Tabelle zusammengestellt.

| Erfindungsgemäße Verbindung | Aufwandmenge (kg/ha) | Wuchshemmwirkung | |
|---|---|---|---|
| | | Gerste | Reis |
| Beispiel 2 | 1,0 | 3 | 3 |
| Beispiel 3 | 1,0 | 4 | 4 |
| Beispiel 8 | 1,0 | 2 | 2 |
| Beispiel 10 | 1,0 | 2 | 4 |
| Beispiel 11 | 1,0 | 2 | 2 |
| Beispiel 12 | 1,0 | 4 | 3 |
| Beispiel 13 | 1,0 | 3 | 2 |
| Vergleichsmittel | | | |
| Mepiquatchlorid + Ethephon | 0,92 | 1 | 0 |

Wie das obige Beispiel zeigt, ist der kommerzielle Standard Mepiquatchlorid + Etephon schwächer

wirksam als die erfindungsgemäßen Substanzen.

## Ansprüche

1. 3,5-Dioxo-4-heteroaroylcyclohexancarbonsäurederivate der allgemeinen Formel I

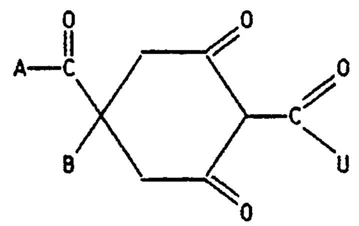

(I) ,

in der
A eine der Gruppen -OM, -$OR^1$ oder -$NR^2R^3$,
B ein Wasserstoffatom, einen $C_1$-$C_8$-Alkylrest, einen $C_2$-$C_8$-Alkenylrest, einan $C_2$-$C_8$-Alkinylrest, einen Halogen-$C_1$-$C_8$-Alkylrest, einen Halogen-$C_2$-$C_8$-Alkenylrest oder einen Halogen-$C_2$-$C_8$-Alkinylrest,
M ein Kation aus der Gruppe Lithium, Natrium und Kalium, ein Äquivalent aus der Gruppe Zink, Mangan, Calcium, Magnesium und Barium oder ein Ammoniumion der allgemeinen Formel

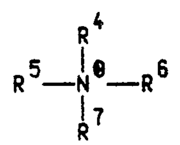

,

$R^1$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein- oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano oder $C_1$-$C_4$-Alkyl substituierten phenylrest, einen Phenyl-$C_1$-$C_2$-alkylrest oder einen ein- oder mehrfach, gleich oder verschieden durch Halogen, Nitro, Cyano oder $C_1$-$C_4$-Alkyl substituierten Phenyl-$C_1$-$C_2$-alkylrest,
$R^2$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein- oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano oder $C_1$-$C_4$-Alkyl substituierten Phenylrest, einen Phenyl-$C_1$-$C_2$-alkylrest oder einen ein- oder mehrfach, gleich oder verschieden durch Halogen, Nitro, Cyano oder $C_1$-$C_4$-Alkyl substituierten Phenyl-$C_1$-$C_2$-alkylrest,
$R^3$ ein Wasserstoffatom, einen $C_1$-$C_{18}$-Alkylrest, einen ein- oder mehrfach, gleich oder verschieden durch Halogen, Hydroxy oder Cyano substituierten oder durch Sauerstoff oder Schwefel ein-oder mehrfach unterbrochenen $C_1$-$C_{18}$-Alkylrest, einen $C_2$-$C_{12}$-Alkenylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkenylrest, einen $C_2$-$C_{12}$-Alkinylrest, einen ein- oder mehrfach, gleich oder verschieden durch Halogen, Hydroxy oder Cyano substituierten oder durch Sauerstoff oder Schwefel ein- oder mehrfach unterbrochenen $C_2$-$C_{12}$-Alkinylrest, einen Phenylrest, einen ein- oder mehrfach, gleich oder verschieden durch Halogen, Nitro, Cyano oder $C_1$-$C_4$-Alkyl substituierten Phenylrest, einen Phenyl-$C_1$-

$C_2$-alkylrest oder einen ein- oder mehrfach, gleich oder verschieden durch Halogen, Nitro, Cyano oder $C_1$-$C_4$-Alkyl substituierten Phenyl-$C_1$-$C_2$-alkylrest oder

$R^2$ und $R^3$ gemeinsam mit dem benachbarten Stickstoffatom eine Morpholinogruppe, eine Piperidinogruppe oder eine Pyrrolidinogruppe,

$R^4$ ein Wasserstoffatom, einem $C_1$-$C_{18}$-Alkylrest, einen ein- oder mehrfach, gleich oder verschieden durch Halogen, Hydroxy, Nitro, Cyano oder $C_1$-$C_6$-Alkoxy substituierten $C_1$-$C_{18}$-Alkylrest, einen Phenylrest, einen ein-oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder ver schieden durch Halogen, Hydroxy, Nitro, Cyano, $C_1$-$C_6$-Alkyl $C_1$-$C_6$-Alkoxy substituierten Phenyl-$C_1$-$C_2$-alkylrest,

$R^5$ ein Wasserstoffatom, einen $C_1$-$C_{18}$-Alkylrest, einen ein- oder mehrfach, gleich oder verschieden durch Halogen, Hydroxy, Nitro, Cyano oder $C_1$-$C_6$-Alkoxy substituierten $C_1$-$C_{18}$-Alkylrest, einen Phenylrest, einen ein-oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenyl-$C_1$-$C_2$-alkylrest,

$R^6$ ein Wasserstoffatom, einen $C_1$-$C_{18}$-Alkylrest, einen ein- oder mehrfach, gleich oder verschieden durch Halogen, Hydroxy, Nitro, Cyano oder $C_1$-$C_6$-Alkoxy substituierten $C_1$-$C_{18}$-Alkylrest, einen Phenylrest, einen ein-oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl $C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenyl-$C_1$-$C_2$-alkylrest,

$R^7$ ein Wasserstoffatom, einen $C_1$-$C_{18}$-Alkylrest, einen ein- oder mehrfach, gleich oder verschieden durch Halogen, Hydroxy, Nitro, Cyano oder $C_1$-$C_6$-Alkoxy substituierten $C_1$-$C_{18}$-Alkylrest, einen Phenylrest, einen ein-oder mehrfach, gleich oder verschieden durch Halogen, Hydroxy, Nitro, Cyano, $C_1$-$C_6$-Alkyl oder $C_1$-$C_6$-Alkoxy substituierten Phenylrest, einen Phenyl-$C_1$-$C_2$-alkylrest oder einen ein- oder mehrfach, gleich oder verschieden durch Halogen, Hydroxy, Nitro, Cyano, $C_1$-$C_6$-Alkyl oder $C_1$-$C_6$-Alkoxy substituierten Phenyl-$C_1$-$C_2$-alkylrest,

U eine der Gruppen U - 1 oder U - 2

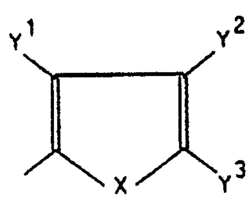

U - 1

oder

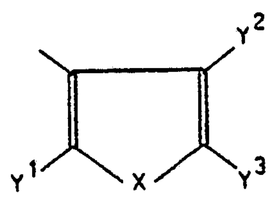

U - 2

,

X ein Sauerstoffatom, ein Schwefelatom oder eine Gruppe N-$R^8$,

$R^8$ ein Wasserstoffatom oder einen $C_1$-$C_4$-Alkylrest,

$Y^1$ ein Wasserstoffatom, einen $C_1$-$C_8$-Alkylrest, einen durch Halogen, $C_1$-$C_8$-Alkoxy, Hydroxy oder Mercapto substituierten $C_1$-$C_8$-Alkylrest, einen $C_2$-$C_8$-Alkenylrest, einen durch Halogen, $C_1$-$C_8$-Alkoxy, Hydroxy oder Mercapto substituierten $C_2$-$C_8$-Alkenylrest, einen $C_2$-$C_8$-Alkinylrest, einen durch Halogen, $C_1$-$C_8$-Alkoxy, Hydroxy oder Mercapto substituierten $C_2$-$C_8$-Alkinylrest, einen $C_1$-$C_8$-Alkoxyrest, einen Halogen-$C_1$-$C_8$-Alkoxyrest, ein Halogenatom, einen Nitrorest, einen Cyanorest, einen Trimethylsilylrest oder eine der Gruppen -COA, -$COR^1$, -$PO(OR^1)_2$ oder -$S(O)_mR^1$,

$Y^2$ ein Wasserstoffatom, einen $C_1$-$C_8$-Alkylrest, einen durch Halogen, $C_1$-$C_8$-Alkoxy, Hydroxy oder Mercapto substituierten $C_1$-$C_8$-Alkylrest, einen $C_2$-$C_8$-Alkenylrest, einen durch Halogen, $C_1$-$C_8$-Alkoxy, Hydroxy oder Mercapto substituierten $C_2$-$C_8$-Alkenylrest, einen $C_2$-$C_8$-Alkinylrest, einen durch Halogen, $C_1$-$C_8$-Alkoxy, Hydroxy oder Mercapto substituierten $C_2$-$C_8$-Alkinylrest, einen $C_1$-$C_8$-Alkoxyrest, einen Halogen-$C_1$-$C_8$-Alkoxyrest, ein Halogenatom, einen Nitrorest, einen Cyanorest, einen Trimethylsilylrest oder eine der Gruppen -COA, -$COR^1$, -$PO(OR^1)_2$ oder -$S(O)_mR^1$,

$Y^3$ ein Wasserstoffatom, einen $C_1$-$C_8$-Alkylrest, einen durch Halogen, $C_1$-$C_8$-Alkoxy, Hydroxy oder Mercapto substituierten $C_1$-$C_8$-Alkylrest, einen $C_2$-$C_8$-Alkenylrest, einen durch Halogen, $C_1$-$C_8$-Alkoxy, Hydroxy oder Mercapto substituierten $C_2$-$C_8$-Alkenylrest, einen $C_2$-$C_8$-Alkinylrest, einen durch Halogen, $C_1$-$C_8$-Alkoxy, Hydroxy oder Mercapto substituierten $C_2$-$C_8$-Alkinylrest, einen $C_1$-$C_8$-Alkoxyrest, einen Halogen-$C_1$-$C_8$-Alkoxyrest, ein Halogenatom, einen Nitrorest, einen Cyanorest, einen Trimethylsilylrest oder eine der

Gruppen -COA, $-COR^1$, $-PO(OR^1)_2$ oder $-S(O)_mR^1$ und
m eine Zahl 0, 1 oder 2
bedeuten, sowie die daraus abgeleiteten Salze der allgemeinen Formel II

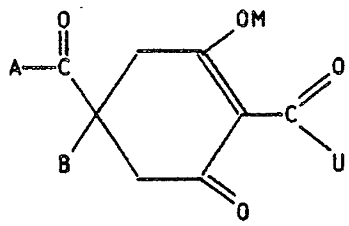

(II) ,

in der A, B, M und U die unter der allgemeinen Formel I genannten Bedeutungen haben.

2. Verfahren zur Herstellung von Verbindung der allgemeinen Formel I ge mäß Anspruch 1, dadurch gekennzeichnet, daß man eine Verbindung der allgemeinen Formel III

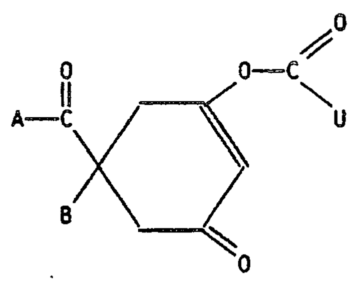

(III) ,

in der A, B und U die unter der allgemeinen Formel I angegebenen Bedeutungen haben, einer katalytisch geförderten Umlagerungsreaktion unterwirft.

3. Mittel mit wachstumsregulierender Wirkung, gekennzeichnet durch einen Gehalt an mindestens einer Verbindung gemäß Anspruch 1.

4. Verwendung von Mitteln gemäß Anspruch 3 zur Wachstumsbeeinflussung von Kulturpflanzen.

5. Verfahren zur Herstellung von Mitteln gemäß Anspruch 3, dadurch gekennzeichnet, daß man eine oder mehrere Verbindungen gemäß Anspruch 1 mit Träger- und/oder sonstigen Hilfsstoffen vermischt.

Europäisches Patentamt

# EUROPÄISCHER RECHERCHENBERICHT

Nummer der Anmeldung

EP 89 11 8201

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,Y | EP-A-0 123 001 (KUMIAI CHEM. IND.)<br>* Insgesamt, und insbesonders Seite 5, Verbindung 44 *<br>--- | 1-5 | C 07 D 307/46<br>C 07 D 333/22<br>C 07 D 207/333<br>A 01 N 43/08<br>A 01 N 43/10<br>A 01 N 43/36 |
| Y | EP-A-0 137 963 (STAUFFER CHEM. COMP.)<br>* Insgesamt *<br>----- | 1-5 | |

| RECHERCHIERTE SACHGEBIETE (Int. Cl.5) |
|---|
| C 07 D 307/00<br>C 07 D 333/00<br>C 07 D 207/00 |

Der vorliegende Recherchenbericht wurde für alle Patentansprüche erstellt

| Recherchenort | Abschlußdatum der Recherche | Prüfer |
|---|---|---|
| DEN HAAG | 04-01-1990 | ALLARD M.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)